# EUROPEAN PATENT SPECIFICATION

(11) **EP 2 253 345 B1**
(45) Date of publication and mention of the grant of the patent: **17.10.2012**
(21) Application number: 09715606.1
(22) Date of filing: 26.02.2009
(51) Int. Cl.: A61M 5/142

(54) **INJECTION DEVICE FOR SEMI-SOLIDIFIED NUTRITIONAL SUPPLEMENT**
INJEKTIONSVORRICHTUNG FÜR HALBFESTE NAHRUNGSERGÄNZUNG
DISPOSITIF D'INJECTION POUR SUPPLÉMENT NUTRITIONNEL SEMI-SOLIDIFIÉ

(30) Priority: 28.02.2008 JP 2008048462
(43) Date of publication of application: 24.11.2010
(73) Proprietor: JMS Co., Ltd., Hiroshima-shi Hiroshima 730-8652 (JP)
(72) Inventor: ITO, Naotsugu, Hiroshima-shi, Hiroshima 730-8652 (JP)
(74) Representative: Stippl, Hubert
(86) International application number: PCT/JP2009/053583
(87) International publication number: WO 2009/107737

(56) References cited:
- EP-A1- 0 300 625
- WO-A1-2007/086186
- JP-A- 4 203 364
- JP-A- 7 313 593
- JP-A- 8 334 087
- JP-A- 2006 304 918
- JP-U- 2 014 169
- US-A- 4 138 205
- US-A- 4 155 362
- US-A- 4 210 138

## Description

### Technical Field

The present invention relates to an injection device that draws a high-viscosity semi-solidified nutritional supplement from a nutritional supplement container and injects it into a patient when performing a nutrition therapy including the direct injection of a nutritional supplement into the stomach, such as a PEG (percutaneous endoscopic gastrostomy) nutrition therapy.

### Background Art

The PEG nutrition therapy is administered to a patient who has difficulty in ingesting food. In the PEG nutrition therapy, a feeding tube is inserted into a fistulous opening of a gastric fistula that is created so as to penetrate through the abdominal wall and the gastric wall of the patient, and then a nutritional supplement is injected into the patient's stomach through the feeding tube. This therapy has been widespread in recent years.

The nutritional supplement is generally a fluid food that is prepared to contain various nutrients such as proteins, carbohydrates, lipid, vitamins, and minerals. The nutritional supplement is prescribed in an amount suitable for the conditions of the patient, and then given to the patient at predetermined time intervals. However, if the nutritional supplement is injected rapidly into the stomach, the patient may have diarrhea or an aspiration pneumonia caused by gastro-esophageal reflux, i.e., the reflux of the nutritional supplement from the stomach into the esophagus. For this reason, it is recommended that the nutritional supplement should be administered somewhat slowly. Therefore, the injection of the nutritional supplement takes a long time, which may increase the burden on a caregiver.

To solve this problem, a method has been proposed that brings the nutritional supplement to an intermediate state between a liquid and a solid, which is called a semi-solidified nutritional supplement, by mixing the nutritional supplement with agar or the like to improve the viscosity, and injects the semi-solidified nutritional supplement. In this method, the nutritional supplement is digested slowly, so that the patient is not likely to suffer from diarrhea. Moreover, since the flowability of the nutritional supplement is low, the patient also has little tendency toward gastro-esophageal reflux. However, due to a high viscosity of the nutritional supplement, it is difficult to inject the semi-solidified nutritional supplement into the stomach via a gastrostomy catheter. The nutritional supplement with a high viscosity has a large resistance as it passes through the tube. Thus, handling of the nutritional supplement involves considerable labor, e.g., the flexible container holding the nutritional supplement has to be pressed by a strong force, or the nutritional supplement has to be put in a special syringe (also referred to as an injector) and injected. This is an impediment to the widespread use of the semi-solidified nutritional supplement.

On the other hand, many types of bag-shaped nutritional supplement containers made of a laminated film have been formed in recent years. In some cases, a liquid nutritional supplement contained in the bag is semi-solidified by the addition of agar or a thickener. Under these circumstances, the nutritional supplement that can be prepared easily as a semi-solidified nutritional supplement, i.e., that can be used quickly is becoming widely available. In a general medical setting, such a semi-solidified nutritional supplement is injected into the patient's stomach, e.g., by pressing strongly a flexible container holding the semi-solidified nutritional supplement with bare hands and extruding the semi-solidified nutritional supplement through the opening of the container.

However, when the semi-solidified nutritional supplement is extruded from the flexible container with the bare hands, since the whole of the flexible container cannot be pressed at a time, the extrusion of nearly all the semi-solidified nutritional supplement is difficult, and thus takes time and effort. Consequently, the semi-solidified nutritional supplement in the container goes to waste, or a long time is required to extrude the semi-solidified nutritional supplement.

To solve the above problem, Patent Document 1 discloses an extruder for a semi-solidified nutritional supplement that includes an air bag (pressure bag) and a receiving member. The air bag is flexible, in the form of a bag, and expands with the supply of air. The receiving member is located opposite to the pressure bag and receives the pressure of the expanded pressure bag. A bag container filled with a semi-solidified nutritional supplement is inserted between the pressure bag and the receiving member, and then the pressure bag is expanded. Thus, due to the cooperation of the pressure bag and the receiving member, the bag container is pressed to force the semi-solidified nutritional supplement out of the bag container. In this manner, the semi-solidified nutritional supplement filled in the bag container can be extruded in a short time compared to the method in which the container is pressed by hand.
Patent Document 1: JP 2007-29562 A

### Disclosure of Invention

### Problem to be Solved by the Invention

However, the method for extruding the semi-solidified nutritional supplement from the bag container using the pressure bag, as disclosed in Patent Document 1, still has the following problems.

First, in the method of Patent Document 1, it is difficult to predict when the flow of the nutritional supplement from the container will finish, and further the nutritional supplement cannot flow out in a short time. Although the flow rate of the nutritional supplement is high at the beginning of pressure application, the flow rate is lower as the remaining amount in the container is reduced, and the nutritional supplement hardly flows after a predetermined period of time. Even if pressure is applied again at the time the flow rate has been low, the same situation is just repeated. Therefore, in order to allow the nutritional supplement to flow in a short time, the pressure has to be applied again by determining the timing at which the flow rate starts to be low. This results in a very complicated operation.

Second, in the method of Patent Document 1, more than a little of the nutritional supplement remains in the container. Therefore, after the nutritional supplement is extruded to some extent with the pressure bag, the bag container is removed from the pressure bag and the receiving member, and the residual liquid needs to be squeezed manually.

To extrude the entire semi-solidified nutritional supplement, e.g., a method has been proposed that transfers the bag container between two rollers so as to squeeze the semi-solidified nutritional supplement by the rotation of the rollers. However, in such a method, the internal pressure of the bag container is increased excessively, which can make it difficult to rotate the rollers. Moreover, depending on the specification of the bag container, a straw is arranged inside the container and therefore interferes with the rotation of the rollers. Thus, the squeezing of the semi-solidified nutritional supplement cannot be completed.

It is an object of the present invention to provide an injection device with a simple structure for a semi-solidified nutritional supplement that easily can extract almost the entire semi-solidified nutritional supplement from a nutritional supplement container and inject it into a patient.

### Means for Solving Problem

An injection device for a semi-solidified nutritional supplement of the present invention injects a semi-solidified nutritional supplement through a pump tube from a nutritional supplement container filled with the semi-solidified nutritional supplement. The injection device includes the following: a base; a rotator that is supported rotatably on the base; a roller that is supported rotatably at the outer periphery of the rotator; and a pressing member that has a circular arc pressing surface along a path of the roller moving in accordance with a rotation of the rotator, is mounted movably on the base, and can be located selectively in a retracted position where the pressing surface is spaced at a distance from the rotator to form a groove-shaped space and in a proximity position where the pressing surface is closer to the rotator than in the retracted position. The pump tube can be inserted into the groove-shaped space when the pressing member is in the retracted position. The pressing member is moved to the proximity position with the pump tube being held, and then the rotator is rotated, so that the pump tube is pressure-driven by the roller.

The injection device of the present invention is useful for the injection of a semi-solidified nutritional supplement. The semi-solidified nutritional supplement in the context of the present invention means both a non-flowable soft food and a flowable high-viscosity food. Specifically, examples of the semi-solidified nutritional supplement include a jelly-like food prepared by mixing a food with agar or the like, a high-viscosity food prepared by adding a thickener or the like to a liquid food (generally having a viscosity of 1000 to 100000 cP (centipoises)), a food prepared with a blender, and a chopped food.

### Effects of the Invention

The injection device for a semi-solidified nutritional supplement of the present invention extracts the semi-solidified nutritional supplement by drawing it from the nutritional supplement container, and thus can reduce the residue in the container sufficiently.

In the conventional method, the nutritional supplement is extruded by pressing the container hard. As a result of studying various methods, the present inventors found out that by squeezing a tube connected to the nutritional supplement container, the semi-solidified nutritional supplement could be extracted easily from the container to the tube in a short time, and that the residue of the nutritional supplement in the container could be reduced significantly.

Based on these findings, the injection device for a semi-solidified nutritional supplement of the present invention realizes a mechanism that is able to squeeze continuously the tube connected to the container holding the semi-solidified nutritional supplement.

with the above configuration, since it is easy to control the semi-solidified nutritional supplement that is squeezed out of the container by the rollers at a constant flow rate based on the number of rotations of the rotator, the injection control can be facilitated.

### Brief Description of Drawings

[FIG. 1] FIG. 1 is a perspective view showing an injection device for a semi-solidified nutritional supplement according to Embodiment 1 of the present invention.
[FIG. 2] FIG. 2 is a perspective view of the injection device in a state different from that shown in FIG. 1.
[FIG. 3] FIG. 3 is a plan view of the injection device in the state of FIG. 2.
[FIG. 4] FIG. 4 is a side view of the injection device in the state of FIG. 2.
[FIG. 5] FIG. 5 is a perspective view showing a state in which a pump tube that is connected to a nutritional supplement container is placed in the injection device in the state of FIG. 1.
[FIG. 6] FIG. 6 is a side view of the injection device in the state of FIG. 1 when viewed from a cover side.
[FIG. 7] FIG. 7 is a perspective view of the injection device in the state of FIG. 1 when viewed from the cover side.
[FIG. 8] FIG. 8 is a perspective view showing a base support for supporting a base of an injection device for a semi-solidified nutritional supplement according to Embodiment 1 of the present invention.
[FIG. 9] FIG. 9 is an enlarged perspective view showing the essential portion of the base support.
[FIG. 10] FIG. 10 is a perspective view showing another example of a base support for supporting a base of an injection device for a semi-solidified nutritional supplement according to Embodiment 1 of the present invention.
[FIG. 11] FIG. 11 shows liquid delivery properties when a semi-solidified nutritional supplement is delivered by a pressure applying method.
[FIG. 12] FIG. 12 shows liquid delivery properties when a semi-solidified nutritional supplement is delivered by an injection device according to Embodiment 1 of the present invention.
[FIG. 13A] FIG. 13A is a plan view showing a part of an injection device for a semi-solidified nutritional supplement according to Embodiment 2 of the present invention.
[FIG. 13B] FIG. 13B is a plan view showing a part of another example of an injection device for a semi-solidified nutritional supplement according to Embodiment 2.
[FIG. 14] FIG. 14 is a perspective view showing a part of yet another example of the configuration of an injection device according to Embodiment 2.
[FIG. 15A] FIG. 15A is a perspective view showing a component of an injection device for a semi-solidified nutritional supplement according to Embodiment 3 of the present invention.
[FIG. 15B] FIG. 15B is an exploded perspective view showing a structure of the component of the injection device.

### Description of Reference Numerals

- 1: Base
- 2: Rotator
- 2a: Radial groove
- 3: Rotator holder
- 4, 34a, 34b: Pressing member
- 4a, 35a: Pressing surface
- 5: Cover
- 5a: Operation hole
- 6: Rotator axis
- 7: Roller
- 7a: Roller axis
- 8: Groove-shaped space
- 9: Cover axis
- 10: Coupling member
- 11: Joint of cover
- 12: Joint of pressing member
- 13a: Entry-side guide pole
- 13b: Exit-side guide pole
- 14a: Entry-side retention groove
- 14b: Exit-side retention groove
- 15: Handle
- 15a: Grip
- 15b: Fitting portion
- 15c: Haft
- 16: Handle holder
- 16a: Upper holding strip
- 16b: Lower holding strip
- 16c: Side strip
- 16d: Grip receiving recess
- 20: Nutritional supplement container
- 21: Pump tube
- 22, 28: Stage
- 23: Drip stand
- 23a: Pole
- 24: Plate
- 25: Bolt
- 26: Stage clamp
- 27: Fastening screw
- 29: Universal arm
- 30: Clamp
- 31, 32, 33: Joint
- 35: Front portion
- 36: Gap
- 37: Supporting portion
- 38a, 38b: Buffer member
- 39: Clutch mechanism
- 40, 41: Fitting axis
- 41a: Upper ratchet gear
- 42: Transmission axis
- 42a: Lower ratchet gear
- 43: Spring

### Best Mode for Carrying Out the Invention

The injection device for a semi-solidified nutritional supplement of the present invention can have the following aspects based on the above configuration.

The injection device further may include the following: a cover that is mounted rotatably on the base and can be located selectively in a seal position where an area above the pressing member and the roller is sealed and in an open position where that area is open; and a coupling member that links a rotation of the cover to a movement of the pressing member. When the cover is in the open position, the pressing member is in the retracted position, and when the cover is in the seal position, the pressing member is in the proximity position.

The injection device further may include a drive mechanism for manually rotating the rotator. In such a case, it is preferable that a rotator axis is provided integrally with the rotator so as to project from a rotator body in its vertical direction, and that a rotator holder is provided on the base and rotatably supports the upper and lower ends of the rotator axis. When the upper and lower ends of the rotator axis are supported rotatably, the operation of pressure-driving the pump tube by the rollers can be stabilized.

It is preferable that the injection device includes a base support for supporting the base.

In the injection device, the cover in the seal position may be configured to expose the upper end of the rotator axis, and a handle can be fitted to the upper end of the rotator axis and can be manipulated to rotate the rotator.

In the injection device, a handle holder for holding the handle may be provided in a position of the base that is on the opposite side of the cover axis to the rotator.

It is preferable that the injection device includes a counter for counting the number of rotations of the rotator.

The counter may have a structure that is operated only by a mechanism.

In the injection device, the pressing member may include a front portion having the pressing surface and a supporting portion that is located on the back of the front portion with a gap interposed between them and connected to the front portion by a buffer member. The buffer member may retract the front portion by an elastic deformation when a pressing force that exceeds a predetermined magnitude is applied via at least the pressing surface.

In the injection device, the rotator may have a driving force transmission member that includes a clutch for receiving a rotational driving force, and when a torque transmitted via the clutch is more than a predetermined value, the clutch is running idle and unable to transmit the driving force.

Moreover, in the injection device, the rotator may have a driving force transmission member that includes a one-way clutch for receiving a rotational driving force, and when a torque transmitted via the one-way clutch is in the opposite direction to a direction in which the pump tube is pressure-driven, the one-way clutch is prevented from revolving or is running idle and unable to transmit the driving force.

Hereinafter, an injection device for a semi-solidified nutritional supplement of an embodiment of the present invention will be described in more detail with reference to the drawings.

### (Embodiment 1)

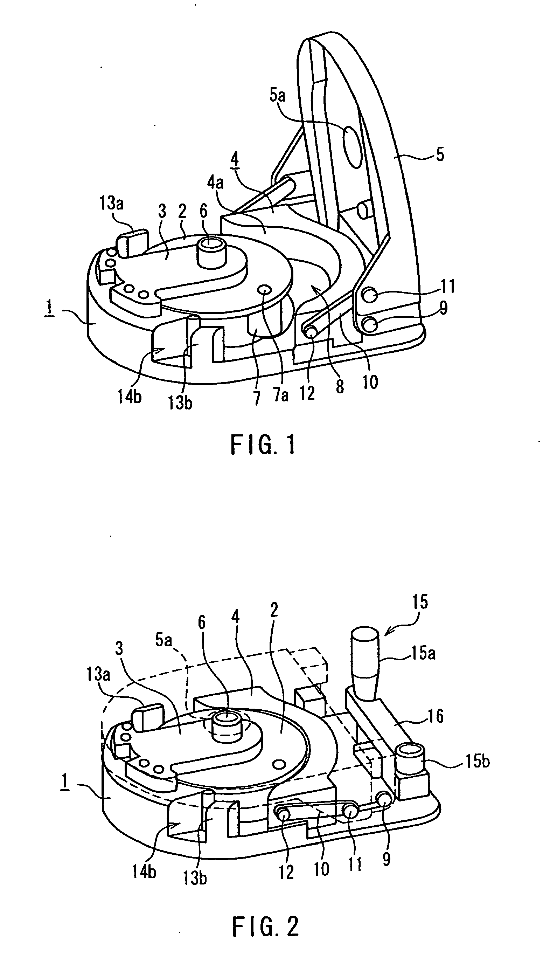

FIG. 1 is a perspective view showing an injection device for a semi-solidified nutritional supplement according to Embodiment 1 of the present invention. The components of the injection device, i.e., a rotator 2, a rotator holder 3, a pressing member 4, and a cover 5 are mounted on a base 1.

The rotator 2 is supported rotatably by the rotator holder 3 that is fixed on the base 1. Therefore, a rotator axis 6 is provided integrally with the rotator 2 and projects in the vertical direction. The upper and lower ends of the rotator axis 6 (the lower end is not shown in the drawing) are supported rotatably by the rotator holder 3. Although the drawing shows only the rotator holder 3 that is located on the upper side of the rotator 2, another rotator holder is located on the lower side of the rotator 2. However, unlike the rotator holder 3 shown in the drawing, the lower rotator holder does not need to be a cantilever in which one end is fixed on the base 1 and the other end is free. In other words, a bearing structure for supporting the lower end of the rotator axis 6 is provided directly on the upper surface of the base 1.

Three rollers 7 are supported rotatably at the outer periphery of the rotator 2. Each of the rollers 7 rotates on its own axis and revolves around the rotator axis 6 in accordance with the rotation of the rotator 2. Therefore, the circular path is formed by the outermost surface of the roller 7 with the rotator axis 6 as the center. The number of the rollers 7 is not limited to three, and two or any other number of rollers also can be used.

The pressing member 4 has a circular arc pressing surface 4a along the path of the rollers 7 moving (rotating) in accordance with the rotation of the rotator 2. The pressing member 4 is mounted movably on the base 1. The relative positional relationship between the pressing surface 4a and the rotator 2 (rollers 7) can be changed between a retracted position (see FIG. 1) where the pressing surface 4a is spaced at a distance from the rotator 2 and a proximity position (see FIG. 2) where the pressing surface 4a is closer to the rotator 2 than in the retracted position. When the pressing member 4 is in the retracted position, as shown in FIG. 1, a wide groove-shaped space 8 is formed between the pressing surface 4a and the rotator 2.

The cover 5 is supported rotatably by a cover axis 9 and can be rotated to take an open position where an area above the pressing member 4 and the rollers 7 is open as shown in FIG. 1 and to take a seal position where that area is sealed (see the broken line in FIG. 2). The cover 5 and the pressing member 4 are coupled by coupling members 10. One end of each of the coupling members 10 is coupled rotatably to a joint 11 of the cover 5, and the other end is coupled rotatably to a joint 12 of the pressing member 4. Therefore, the pressing member 4 moves simultaneously as the cover 5 is rotated via the coupling members 10. Specifically, when the cover 5 is in the open position, the pressing member 4 is in the retracted position, and when the cover 5 is in the seal position, the pressing member 4 is in the proximity position.

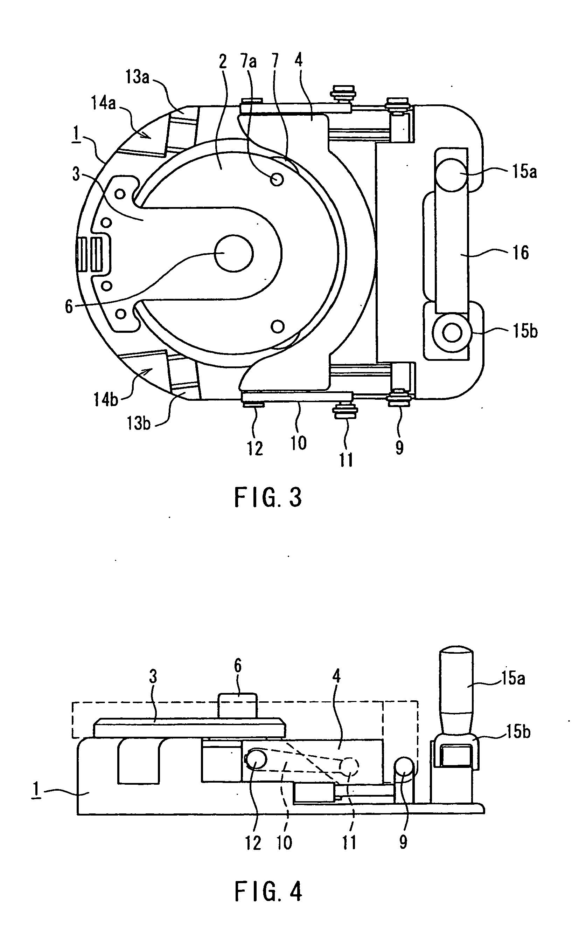

FIG. 2 is a perspective view showing a state in which the cover 5 is in the seal position, and therefore the pressing member 4 is in the proximity position. FIG. 3 and FIG. 4 show a plan view and a side view of this state, respectively. For improved understanding, the cover 5 is represented by the broken line in FIGs. 2 and 4, and is omitted in FIG. 3.

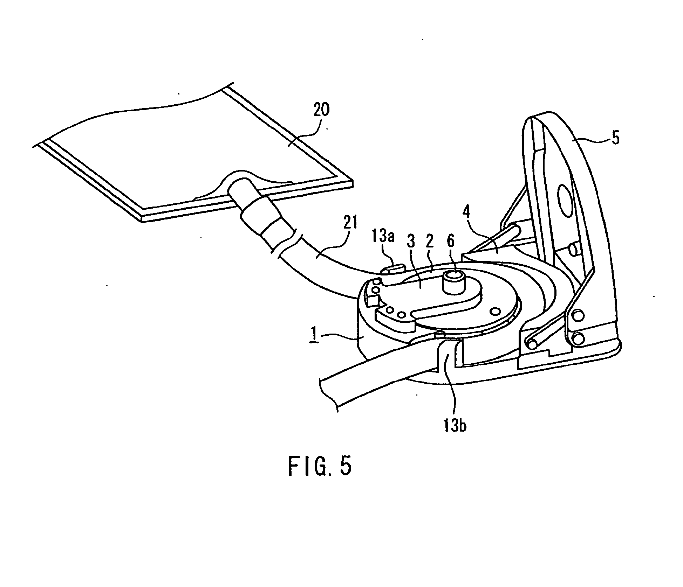

FIG. 5 is a perspective view showing a state in which a pump tube 21 that is connected to a nutritional supplement container 20 filled with a semi-solidified nutritional supplement is placed in the injection device of FIG. 1. The pump tube 21 is inserted into the groove-shaped space 8 between the pressing surface 4a and the rotator 2 in FIG. 1. Thus, the pump tube 21 can be inserted into the groove-shaped space 8 that is formed when the pressing member 4 is in the retracted position. The base 1 has an entry-side guide pole13a and an exit-side guide pole 13b that are opposite to the ends of the pressing member 4, respectively As shown in FIG. 5, the entry side of the inserted pump tube 21 is held in an entry-side retention groove 14a (see FIG. 3) formed by the entry-side guide pole 13a. The exit side of the pump tube 21, i.e., the delivery side used to inject the semi-solidified nutritional supplement is held in an exit-side retention groove 14b formed by the exit-side guide pole 13b.

As shown in FIGs. 2 to 4, when the pressing member 4 is in the proximity position, the lumen of the pump tube 21 is compressed between the pressing member 4 and each of the rollers 7. At the location between two adjacent rollers 7, i.e., the location where the pressing member 4 does not face the roller 7, the pump tube 21 comes into the internal space of the rotator 2, and the lumen of the tube is released.

By rotating the rotator 2 while the pressing member 4 is in the proximity position, and the lumen of the pump tube 21 is compressed between the pressing member 4 and each of the rollers 7, the pump tube 21 can be pressure-driven by the rollers 7. That is, the pump tube 21 is squeezed by the rollers 7, and thus the semi-solidified nutritional supplement is drawn from the nutritional supplement container 20 to the delivery side.

The pump tube 21 is inserted into the groove-shaped space 8 when the pressing member 4 is in the retracted position, and then the cover 5 is rotated to the seal position so as to move the pressing member 4 to the proximity position, thereby covering the area above the pump tube 21. This can prevent the operator or patient from inadvertently touching the rotating bodies (such as the rotator and the rollers) of the pump.

Next, a drive mechanism for manually rotating the rotator 2 will be described. In this embodiment, the cover 5 in the seal position is configured to expose the upper end of the rotator axis 6. As shown in FIG. 1, the cover 5 has an operation hole 5a in the center. The operation hole 5a coincides with the position of the rotator axis 6 when the cover 5 is in the seal position, as shown in FIGs. 2 and 4. Therefore, the upper end of the rotator axis 6 passes through the operation hole 5a and is exposed.

Moreover, the injection device of this embodiment includes a handle 15 for the manual operation to rotate the rotator 2. The handle 15 has a structure such that a grip 15a and a fitting portion 15b are provided at both ends of a haft (not shown in FIGs. 1 to 4). The fitting portion 15b is fitted to the upper end of the rotator axis 6, and then the grip 15a is manipulated, so that a rotational driving force can be transmitted to the rotator axis 6. The base 1 is provided with a handle holder 16, and the handle 15 is held on the base 1 by putting the haft of the handle 15 into the handle holder 16.

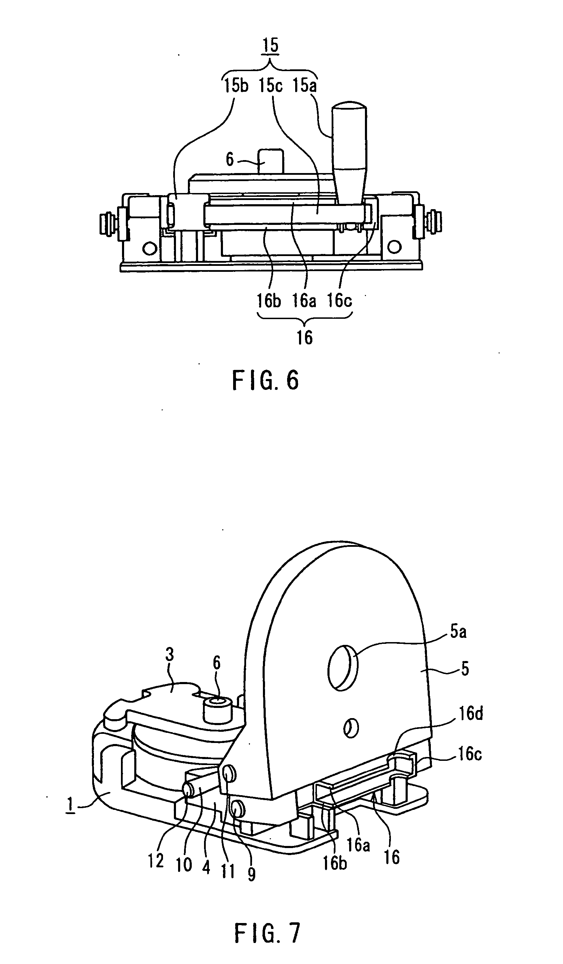

The structures of the handle 15 and the handle holder 16 will be described with reference to FIGs. 6 and 7 in addition to the above drawings. FIG. 6 is a side view of the injection device in FIG. 1 when viewed from the cover 5 side, and the representation of the cover 5 is omitted. FIG. 7 is a perspective view of the injection device in FIG. 1 when viewed from the cover 5 side, and the handle 15 is removed from the handle holder 16. As shown in FIG. 7, the cover 5 can be located in the open position only when the handle 15 is removed from the handle holder 16.

As described above, the handle holder 16 is provided on the opposite side of the cover 5 to the rotator 2 on the base 1. Therefore, when the cover 5 is being rotated to the open position, the handle 15 is held in the position that interferes with the rotation of the cover 5. However, in such circumstances, the handle 15 is used and removed, so that the cover 5 can be rotated without a hitch. On the other hand, after the injection of the semi-solidified nutritional supplement by the injection device is finished, the cover 5 is rotated to the seal position. Thus, there is no hindrance to the insertion of the handle 15 into the handle holder 16.

FIG. 6 shows the haft 15c of the handle 15. The handle holder 16 includes an upper holding strip 16a, a lower holding strip 16b, and a side strip 16c, as shown in FIG. 7. A grip receiving recess 16d, which is a circular arc cut, is formed at the end of the upper holding strip 16a near the side strip 16c. As shown in FIG. 7, when the haft 15c of the handle 15 is sandwiched between the upper and lower holding strips 16a, 16b, the handle holder 16 holds the handle 15 by the haft 15c. In this state, the root of the grip 15a is placed in the grip receiving recess 16d, and the fitting portion 15b is positioned extending from the left end area between the upper holding strip 16a and the lower holding strip 16b.

The handle holder 16 also can be used as a handle to carry the whole injection device with the cover 5 located in the seal position. Moreover, although not shown, the handle 15 may have a foldable structure. In the case of the handle 15 with the foldable structure, when attached on the upper surface of the rotator, one end of the handle is raised from a folded state and held by a hand, so that the rotator can be rotated with a small force. The handle is folded back out of the way when the rotator is not rotated. Even in a state in which the handle 15 is folded, it can be fitted in the rotator 2 when the cover 5 is in the seal position, and also can be operable for rotation.

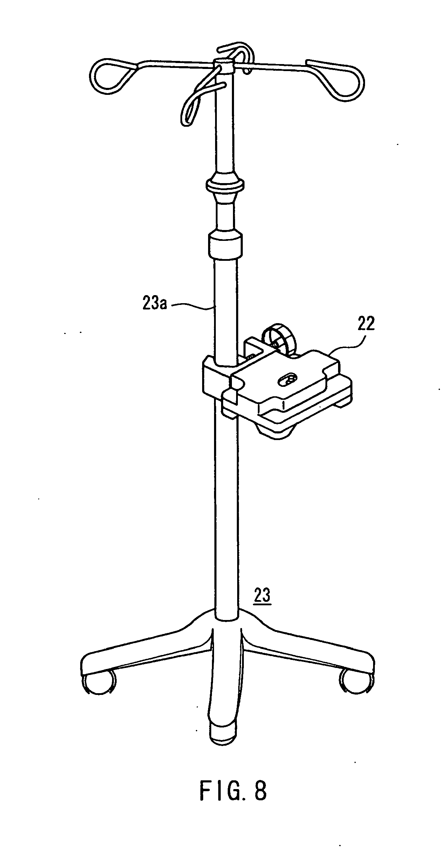

FIG. 8 shows an example of a base support for supporting the base 1 of the injection device of the present invention. By attaching the base 1 to this base support, the injection device can be held stably, and the handle 15 can be manipulated very easily to rotate the rotator 2. The base support is configured by combining a stage 22 provided with a clamp and a drip stand 23. The stage 22 has a structure detachable from the base 1.

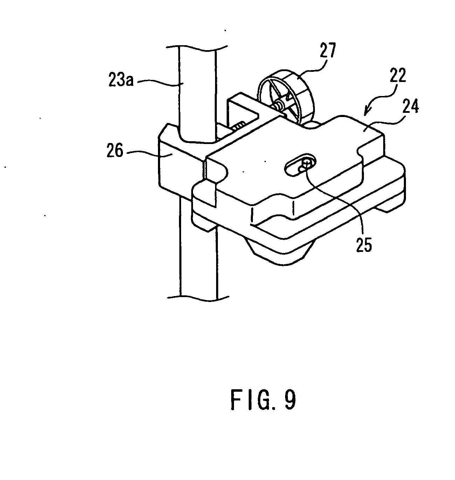

As shown in the enlarged view of FIG. 9, the stage 22 has a plate 24 with a flat surface that supports the base 1. There is a hole in the center of the plate 24, from which the end of a bolt 25 for clamping the base 1 projects. Although not shown, a threaded hole that receives the bolt 25 is formed in the lower surface of the base 1, and the base 1 is fixed on the plate 24 by the bolt 25. The stage 22 further has a stage clamp 26 and a fastening screw 27. A pole 23a of the drip stand 23 is put in the stage clamp 26 and fixed by the fastening screw 27.

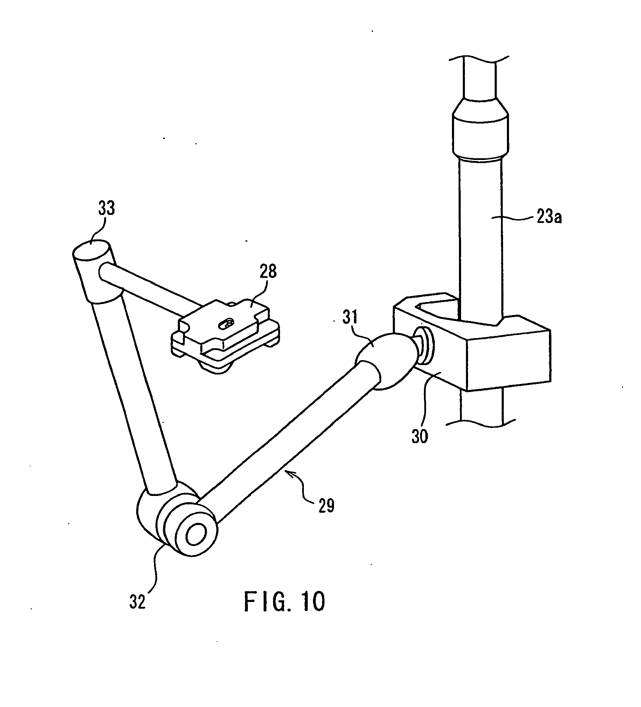

FIG. 10 shows another example of the base support. This base support includes a universal arm 29 that is interposed between a stage 28 provided with a clamp and a drip stand 23, which are similar to those shown in FIG. 8. The universal arm 29 has a clamp 30 that is fixed to the pole 23a of the drip stand 23. The end of the universal arm 29 is connected to the stage 28. The universal arm 29 can be bent at intermediate joints 31, 32, and 33 and has the general function of being able to adjust the position and posture of the stage 28 flexible

As the base support, any structure other than the stages 22, 28 provided with a clamp can be selected as desired. For example, one of hook-and-loop fasteners may be fixed to the bottom of the base 1 and the other may be fixed on a table sheet. In such a case, a sufficient support effect cannot be obtained if the table sheet is smaller than the base 1. Therefore, the area of the table sheet is preferably at least two times, and more preferably at least four times as large as the area of the base 1. By using the base support, the position and direction of the injection device can be changed, and thus the semi-solidified nutritional supplement container can be hung with its outlet facing down. Consequently, a blockage is not likely to occur at the end of the container and, even if the blockage occurs, it can be removed quickly.

In the injection device for a semi-solidified nutritional supplement based on the above configurations and operations, the semi-solidified nutritional supplement is drawn from the container and leaves almost no residue in the container. Similarly, the residue in the lumen of the pump tube 21 also can be reduced sufficiently

Since it is easy to control the amount of injection of the semi-solidified nutritional supplement that is squeezed out of the container by the rollers 7, the injection control can be facilitated. That is, the amount of injection of the semi-solidified nutritional supplement corresponds to the number of rotations (the times of rotation) of the rotator 2. Therefore, the amount of injection can be known from the number of rotations. For this reason, although not shown, it is desirable that a counter for counting the number of rotations of the rotator 2 be provided. The use of the counter makes it easy to control the amount of injection. Moreover, even if the rotator 2 is rotated manually, the counter can be used to adjust the amount of injection of the semi-solidified nutritional supplement.

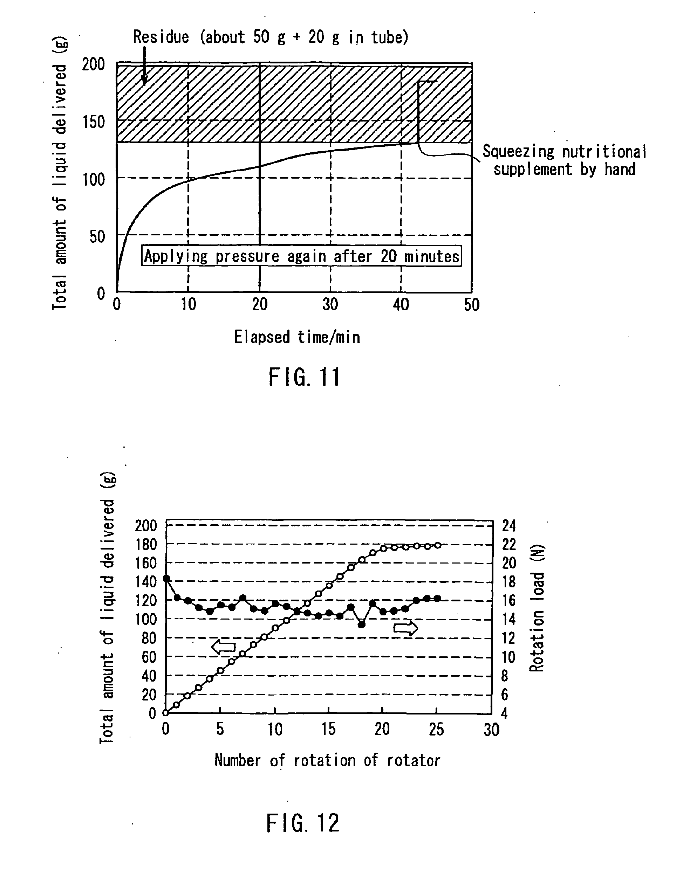

The ease of control of the amount of injection of the semi-solidified nutritional supplement in the injection device of this embodiment will be described with reference to FIGs. 11 and 12. FIG. 11 shows the liquid delivery properties when the semi-solidified nutritional supplement is delivered by the pressure applying method as described in the Background Art. The horizontal axis indicates the elapsed time from the start of the delivery of the liquid, and the vertical axis indicates the total amount of liquid delivered. FIG. 12 shows the liquid delivery properties when the semi-solidified nutritional supplement is delivered by the injection device of this embodiment. The horizontal axis indicates the number of rotations of the rotator from the start of the delivery of the liquid. The left vertical axis indicates the total amount of liquid delivered and the right vertical axis indicates the rotation load. The line graph with white circles (O) indicates the total amount of liquid delivered, and the line graph with black circles (●) indicates the rotation load.

In the case of the pressure applying method in FIG. 11, first pressure is applied, and then the container is allowed to stand while the nutritional supplement is delivered. Subsequently, pressure is applied again after 20 minutes, and the nutritional supplement continues to be delivered and finally is squeezed out of the container by hand. As can be seen from FIG. 11, the amount of liquid delivered significantly changes with time, making it difficult to control the desired total amount of liquid delivered. Moreover, it is evident that a large amount of the semi-solidified nutritional supplement has not been injected.

On the contrary, in the case of the injection device of this embodiment in FIG. 12, the flow rate is substantially constant with respect to the number of rotations of the rotator. Therefore, the total amount of liquid delivered changes linearly with the number of rotations of the rotator, making it easy to control the desired total amount of liquid delivered. Thus, if the number of rotations of the rotator is controlled by a counter, the desired total amount of liquid delivered can be achieved easily. Moreover, the rotation load changes little with the number of rotations of the rotator, and the operation can be continued stably under controlled conditions.

When the injection device of this embodiment in FIG. 12 is operated manually, even if the rotator is rotated at a slow speed so that it takes 2 to 3 seconds for one revolution, the liquid delivery of almost the entire amount of the bag requires only about one minute (50 to 75 seconds). Accordingly, the treatment time can be reduced compared to the injection device using the pressure applying method, in which 25% or more of residue is left after 30 to 40 minutes of operation.

The counter may be either mechanical or electrical. However, when the injection means of the semi-solidified nutritional supplement is a manual pump, the counter is preferably mechanical in view of its portability and use in a place where the electric power is not supplied.

In the configuration of this embodiment, the semi-solidified nutritional supplement can be injected efficiently by a simple operation that only requires inserting the pump tube 21, rotating the cover 5 to move the pressing member 4, and subsequently rotating the rotator 2 with the handle 15. A large force is not required to rotate the handle 15. Moreover, unlike the method for squeezing the semi-solidified nutritional supplement out of the container by the rotation of the roller, the same effect can be obtained by operating the injection device in the same manner regardless of the structure of the nutritional supplement container. Further, the injection device has a simple and strong structure.

In the above embodiment, the rotator 2 is rotated manually with the handle 15. However, the rotator 2 also may be rotated mechanically with a motor or the like. In such a case, the same effect as described above can be obtained.

The pump tube used with the injection device of the present invention can be the same as the tube that is made of a flexible synthetic resin and used for an infusion pump or a blood pump. Preferred materials include a flexible polyvinyl chloride resin, a polybutadiene resin, a flexible polypropylene resin, and a silicone resin. In particular, it is preferable to use a material such that the additive of the material such as a plasticizer is not eluted or is eluted in the smallest possible amount when the material comes into contact with the nutritional supplement.

It is also preferable that a junction means such as a connector is provided at the ends of the pump tube so as to be able to be connected with the nutritional supplement container and a gastrostomy catheter. The tube preferably has an inner diameter of 2 to 10 mm and a wall thickness of 1.0 to 3.0 mm.

### (Embodiment 2)

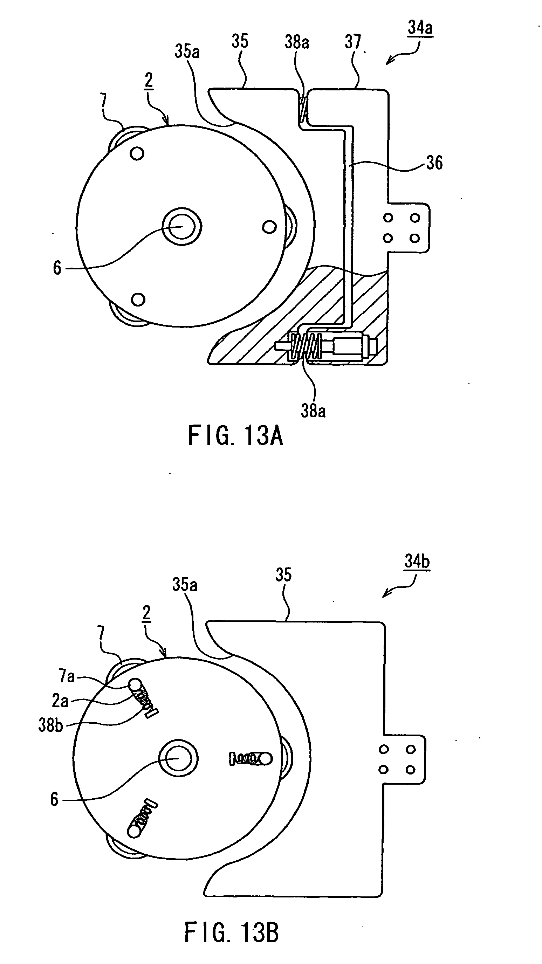

FIG. 13A is a plan view showing a part of an injection device for a semi-solidified nutritional supplement according to Embodiment 2. In FIG. 13A, the same components as those of Embodiment 1 shown in FIG. 3 are denoted by the same reference numerals, and the explanation will not be repeated. The operations using the same components as those of Embodiment 1 will be described with reference to FIGs.1 to 5 as well.

FIG. 13A only shows a rotator 2 and a pressing member 34a (corresponding to the rotator 2 and the pressing member 4 in FIG. 3) of the components of the injection device. This embodiment is the same as Embodiment 1 in configuration except that the structure of the pressing member 34a differs from that of the pressing member 4 of Embodiment 1. The pressing member 34a is shown partially in cross section.

The pressing member 34a is divided into a front portion 35 having a pressing surface 35a and a supporting portion 37 that is located on the back of the front portion 35 with a gap 36 interposed between them. The supporting portion 37 and the front portion 35 are connected by buffer members 38a. Although not shown in FIG. 13A, the supporting portion 37 is joined to the coupling members 10 in the same structure as shown in FIG. 1, and thus can be coupled to the cover 5 via the coupling members 10.

The buffer members 38a may be in the form of a spring so as to be deformable elastically. Therefore, the front portion 35 can be moved due to the elastic deformation of the buffer members 38a in the direction in which the size of the gap 36 between the front portion 35 and the supporting portion 37 is changed. In other words, the size of the groove-shaped space 8 (see FIG. 1) between the pressing surface 35a and the rotator 2 is changed by the elastic deformation of the buffer members 38a.

When the pump tube 21 (see FIG. 5) is pressure-driven by the rollers 7 in accordance with the rotation of the rotator 2, the internal pressure of the pump tube 21 is increased to press the pump tube 21 against the pressing surface 35a. If the internal pressure of the pump tube 21 is within a normal range, the pressing force that acts on the pressing surface 35a will not be large enough to deform the buffer members 38a. Thus, the pressing member 34a is operated in the same manner as the pressing member 4 described in Embodiment 1.

On the other hand, if the internal pressure of the pump tube 21 is increased beyond the normal range, the pressing surface 35a receives the pressing force that exceeds a predetermined magnitude due to the expansion of the pump tube 21. Consequently, the buffer members 38a are deformed elastically to retract the front portion 35, i.e., the pressing surface 35a. This widens the groove-shaped space 8 between the pressing surface 35a and the rotator 2. In other words, if the internal pressure of the pump tube 21 is increased excessively, the retraction of the pressing surface 35a can provide the effect of relieving the internal pressure of the pump tube 21.

Because of the above effect, this embodiment can avoid the following problems. For example, if a blockage occurs in the downstream portion of the pump tube 21 when the rollers 7 continue to drive the pump tube 21 by the rotation of the rotator 2, the internal pressure of the pump tube 21 is increased excessively This may cause a disconnection or breakage of the pump tube 21. In contrast, when the internal pressure of the pump tube 21 is relieved by the retraction of the pressing surface 35a, such an accident can be avoided.

In the above configuration, the buffer members are provided in the pressing member of the pump to relieve an excessive increase in the internal pressure of the pump tube 21 due to pressure driving by the rollers 7 of the rotator 2. However, the buffer members may be provided in the rotator 2 or the rollers 7.

For example, as shown in FIG. 13B, a pressing member 34b is formed integrally, and the pressing surface 35a is fixed. On the other hand, three rollers 7 are not fixed to the rotator 2, but the roller axes 7a are inserted into radial grooves 2a of the rotator 2 and supported movably in the radial direction. The roller axes 7a are biased toward the outer circumference of the rotator 2 by spring-like buffer members 38b. The buffer members 38b are designed to retract the rollers 7 toward the center of the rotator 2 by the elastic deformation when the pressing force that exceeds a predetermined magnitude is applied via at least the pressing surface 35a. This configuration can have substantially the same effect as that obtained by providing the buffer members in the pressing member.

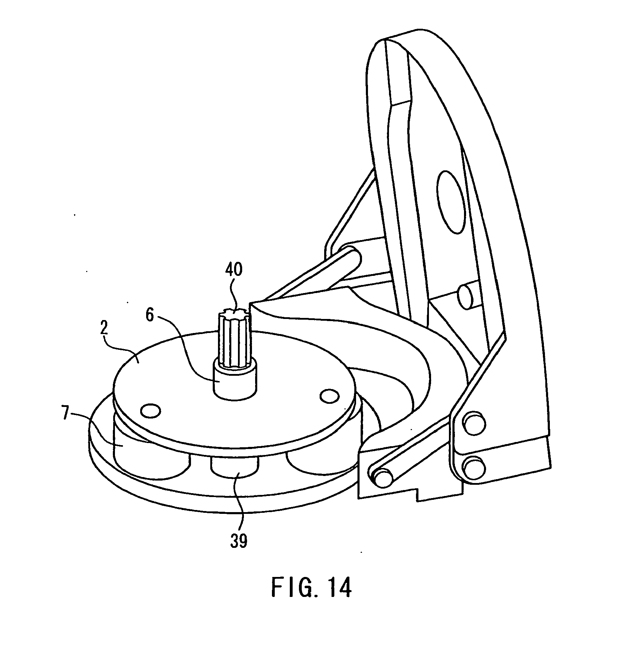

Further, to relieve an excessive increase in the internal pressure of the pump tube 21 due to pressure driving by the rollers 7 of the rotator 2, another configuration shown in FIG. 14 also can be used instead of the configurations shown in FIGs.13A and 13B.

FIG. 14 only shows a rotator 2, a pressing member 4, and a cover 5 of the components of the injection device of this embodiment (the base 1 shown in FIG. 1 is removed). This configuration differs from Embodiment 1 in that a clutch is included inside the rotator axis 6 as a driving force transmission member for transmitting a driving force to rotate the rotator 2. A clutch mechanism 39 is provided in the lower portion of the rotator axis 6, and a fitting axis 40 that transmits a driving force to the clutch projects upward from the rotator axis 6.

The fitting axis 40 is rotatable with respect to the rotator axis 6 and can be joined integrally with the rotator 2 via the clutch in the clutch mechanism 39. Therefore, the rotational driving force that is applied to the fitting axis 40, e.g., by the handle 15 rotates the rotator 2 via the clutch.

However, if the torque transmitted to the clutch is more than a predetermined value, the clutch is running idle and unable to transmit the driving force. Therefore, as described above, if the rollers 7 continue to drive the pump tube 21 while the downstream portion of the pump tube 21 is blocked, and consequently the internal pressure of the pump tube 21 is increased excessively, the clutch is running idle and the driving force is not transmitted, so that the rotator 2 is stopped. Thus, an increase in the internal pressure of the pump tube 21 can be suppressed, thereby avoiding an accident.

A mechanism for idling the clutch when the transmitted torque is more than a predetermined value can be, e.g., a structure of a general torque driver.

The clutch mechanism 39 may be used with the structure in which the front portion 35 and the supporting portion 37 of the pressing member 34a are connected by the buffer members 38a, as shown in FIG. 13A.

### (Embodiment 3)

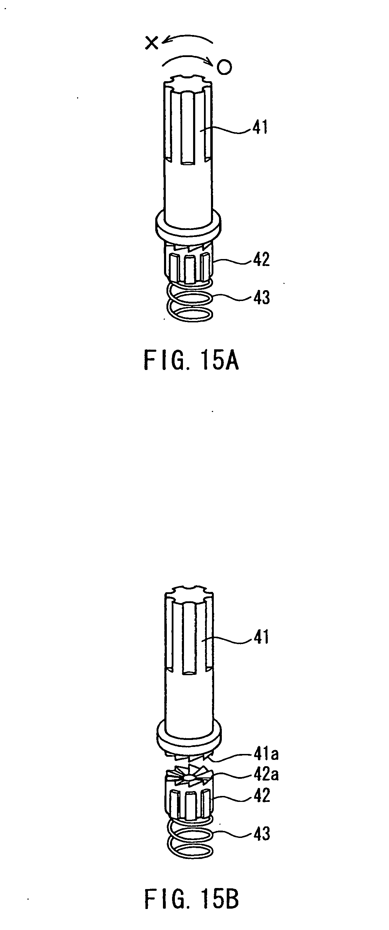

FIG. 15A is a perspective view showing the essential portion of an injection device for a semi-solidified nutritional supplement according to Embodiment 3. FIG. 15B is an exploded view showing a structure of the essential portion. The whole configuration of the injection device of this embodiment is the same as that of Embodiment 1. The rotator 2 has a similar structure to that shown in FIG. 14 of Embodiment 2.

In this embodiment, the clutch mechanism 39 shown in FIG. 14 is slightly different from Embodiment 2 and serves as a one-way clutch for transmitting a driving force to the rotator 2. FIG. 15A shows an example of the one-way clutch. FIG. 15B is an exploded view of the one-way clutch.

The one-way clutch includes an upper ratchet gear 41a that is located at the lower end of a fitting axis 41 and a lower ratchet gear 42a that is provided on a transmission axis 42 and engaged with the upper ratchet gear 41a. The transmission axis 42 is engaged with the inner wall of the clutch mechanism 39 so that it cannot be rotated, but moved in the axial direction. Moreover, the transmission axis 42 is biased by a spring 43 in the direction in which the upper ratchet gear 41a comes into contact with the lower ratchet gear 42a.

Therefore, although the clockwise rotation of the fitting axis 41 is transmitted to the transmission axis 42, the counterclockwise rotation is not transmitted, providing a function of preventing reverse rotation. Thus, if the torque transmitted to the one-way clutch is in the opposite direction to the direction in which the pump tube 21 (see FIG. 5) is pressure-driven, the one-way clutch is running idle and unable to transmit the driving force.

Because of the above effect, this embodiment can avoid the following problems. If the rollers 7 of the rotator 2 are rotated in the reverse direction during the injection of the semi-solidified nutritional supplement, a negative pressure is produced in the internal cavity of the stomach of the patient under treatment, and may cause pain to the patient. Thus, if the function of preventing reverse rotation is provided, as in the case of this embodiment, such a troublesome situation can be avoided.

### Industrial Applicability

The present invention can provide an injection device that can extract a semi-solidified nutritional supplement filled in a nutritional supplement container with a small force, in a short time, and until the residue in the container is reduced sufficiently, and can inject it into a patient. Thus, the present invention is useful for an injection device used for a tube feeding therapy such as a PEG therapy.

## Claims

1. An injection device for a semi-solidified nutritional supplement that injects a semi-solidified nutritional supplement through a pump tube (21) from a nutritional supplement container filled with the semi-solidified nutritional supplement, comprising:
a base (1);
a rotator (2) that is supported rotatably on the base (1);
a roller (7) that is supported rotatably at an outer periphery of the rotator (2);
a pressing member (4) that has a circular arc pressing surface (4a) along a path of the roller (7) moving in accordance with a rotation of the rotator (2), is mounted movably on the base (1), and can be located selectively in a retracted position where the pressing surface (4a) is spaced at a distance from the rotator (2) to form a groove-shaped space (8) and in a proximity position where the pressing surface (4a) is closer to the rotator (2) than in the retracted position,
a cover (5) that is mounted rotatably on the base (1) and can be located selectively in a seal position where an area above the pressing member (4) and the roller (7) is sealed and in an open position where that area is open; and
a coupling member (10) that links a rotation of the cover (5) to a movement of the pressing member (4), so that when the cover (5) is in the open position, the pressing member (4) is in the retracted position, and when the cover (5) is in the seal position, the pressing member (4) is in the proximity position.
wherein the pump tube (21) can be inserted into the groove-shaped space (8) when the pressing member (4) is in the retracted position, and the pressing member (4) is moved to the proximity position with the pump tube (21) being held, and then the rotator (2) is rotated, so that the pump tube (21) is pressure-driven by the roller (7),
**characterized in that**
a rotator axis (6) is provided integrally with the rotator (2) so as to project from a rotator body in its vertical direction,
a rotator holder (3) is provided on the base (1) and rotatably supports upper and lower ends of the rotator axis (6), and
the cover (5) has an operation hole (5a) that coincides with the position of the rotator axis (6) when the cover (5) is in the seal position, so that an upper end of the rotator axis (6) passes through the operation hole (5a) and is exposed, enabling a handle (15) to be fitted to the upper end of the rotator axis (6) through the operation hole (5a) and manipulated to rotate the rotator (2).

2. The injection device according to claim 1, further comprising a base (1) support for supporting the base (1).

3. The injection device according to claim 1, further comprising a base support for supporting the base so as not to be unstable when the rotator (2) is rotated manually.

4. The injection device according to any one of claims 1 to 3, further comprising a counter for counting the number of rotations of the rotator.

5. The injection device according to claim 4, wherein the counter has a structure that is operated only by a mechanism.

6. The injection device according to claim 1, wherein the pressing member comprises a front portion having the pressing surface (35a) and a supporting portion (37) (4) that is located on a back of the front portion (35) with a gap (36) interposed between them and connected to the front portion (35) by a buffer member (38a), and
the buffer member (38a) retracts the front portion (35) by an elastic deformation when a pressing force that exceeds a predetermined magnitude is applied via at least the pressing surface (35a).

7. The injection device according to claim 1, wherein the roller (7) is supported at the outer periphery of the rotator (2) so that a rotation axis of the roller (7) is movable in a radial direction of the rotator (2), and is biased toward an outer circumference of the rotator (2) by a buffer member (38b), and
the buffer member (38b) retracts the roller (7) toward a center of the rotator (2) by an elastic deformation when a pressing force that exceeds a predetermined magnitude is applied via at least the pressing surface.

8. The injection device according to claim 1, wherein the rotator (2) has a driving force transmission member that includes a clutch for receiving a rotational driving force, and
when a torque transmitted via the clutch is more than a predetermined value, the clutch is running idle and unable to transmit the driving force.

9. The injection device according to claim 1, wherein the rotator (2) has a driving force transmission member that includes a one-way clutch for receiving a rotational driving force, and
when a torque transmitted via the one-way clutch is in an opposite direction to a direction in which the pump tube is pressure-driven, the one-way clutch is prevented from revolving or is running idle and unable to transmit the driving force.

## Patentansprüche

1. Zuführvorrichtung für einen halbverfestigten Ernährungszusatz, der aus einem Behälter durch einen Pumpenschlauch (21) zugeführt wird, mit
einem Basisteil (1),
einem drehbar auf diesem gelagerten Drehteil (2),
einer drehbar am Außenumfang des Drehteils (2) gelagerten Rolle (7),
einem Andruckteil (4), das eine kreisbogenförmige Andruckfläche (4a) längs des Weges der Rolle (7) bei deren Bewegung mit der Drehung des Drehteils (2) aufweist und beweglich an dem Basisteil (1) befestigt ist und wahlweise in eine Rückzugs-Position bewegbar ist, in welcher die Andruckfläche (4a) unter Bildung eines ringförmigen Zwischenraums (8) einen Abstand zum Drehteil (2) hat, und in eine Nah-Position bewegbar ist, in welcher sich die Andruckfläche (4a) näher bei dem Drehteil (2) als in der Rückzugs-Position befindet,
einem Deckelteil (5), welches drehbar auf dem Basisteil (1) montiert ist und wahlweise in eine Verschlussposition bringbar ist, in welcher ein Bereich oberhalb des Andruckteils (4) und der Rolle (7) verschlossen ist, und in eine offene Position bringbar ist, in welcher dieser Bereich offen ist, und
einem Kupplungsteil (10), welches die Bewegung des Deckelteils (5) mit einer Bewegung des Andruckteils (4) kuppelt, sodass bei offener Position des Deckelteils (5) sich das Andruckteil (4) in der Rückzugs-Position befindet und bei geschlossenem Deckel (5) das Andruckteil (4) sich in der Nahposition befindet,
und der Pumpenschlauch (21) in den rillenförmigen Zwischenraum (8) einbringbar ist, wenn sich das Andruckteil (4) in der Rückzugs-Position befindet, und das Andruckteil (4) dann in die Nahposition bewegt wird und den Pumpenschlauch (21) festhält, und dann das Drehteil (2) gedreht wird, sodass der Pumpenschlauch (21) von der Rolle (7) mit Druck beaufschlagt wird,
**dadurch gekennzeichnet,**
**dass** eine mit dem Drehteil (2) einteilige Drehteilachse (6) vorgesehen ist, welche von dem Drehteilkörper in vertikaler Richtung wegragt,
**dass** am Basisteil (1) ein Drehteilhalter (3) vorgesehen ist, in welchem die oberen und unteren Enden der Drehteilachse (6) drehbar gelagert sind,
**dass** das Deckelteil (5) eine Antriebsöffnung (5a) aufweist, die mit der Position der Drehteilachse (6) übereinstimmt, wenn sich das Deckelteil (5) in der Verschlussposition befindet, wobei das obere Ende der Drehteilachse (6) durch die Antriebsöffnung (5a) ragt und frei liegt, sodass ein Handgriff (15) durch die Antriebsöffnung (5a) an das obere Ende der Drehteilachse (6) ansetzbar ist und zum Drehen des Drehteils (2) betätigt werden kann.

2. Zuführvorrichtung nach Anspruch 1, weiterhin mit einem Basisteilträger zur Halterung des Basisteils (1).

3. Zuführvorrichtung nach Anspruch 1, weiterhin mit einem Basisteilträger zur Halterung des Basisteils derart, dass dieses nicht unstabil ist, wenn das Drehteil (2) von Hand gedreht wird.

4. Zuführvorrichtung nach einem der Ansprüche 1-3, weiterhin mit einem Zähler zum Zählen der Anzahl von Umdrehungen des Drehteils.

5. Zuführvorrichtung nach Anspruch 4, bei welcher der Zähler so gebaut ist, dass er nur durch einen Mechanismus betrieben wird.

6. Zuführvorrichtung nach Anspruch 1, bei welcher das Andruckteil einen Vorderteil mit der Andruckfläche (35a) und einen Halterungsteil (37) (4) hat, der sich unter Bildung eines Zwischenraums (26) an der Rückseite des Vorderteils (5) befindet und mit dem Vorderteil (35) durch ein Pufferteil (38a) verbunden ist, welches unter elastischer Verformung das Vorderteil (35) zurückzieht, wenn eine eine vorbestimmte Größte überschreitende Druckkraft über mindestens die Andruckfläche (35a) zugeführt wird.

7. Zuführvorrichtung nach Anspruch 1, bei welcher die Rolle (7) am Außenumfang des Drehteils (2) gelagert ist, sodass die Drehachse der Rolle (7) in radialer Richtung des Drehteils (2) bewegbar ist und durch ein Pufferteil (38b) gegen einen Außenumfang des Drehteils (2) gedrückt wird,
und wobei das Pufferteil (38b) unter elastischer Verformung die Rolle (7) zur Mitte des Drehteils (2) zurückzieht, wenn die über mindestens die Andruckfläche zugeführte Kraft eine vorbestimmte Größe überschreitet.

8. Zuführvorrichtung nach Anspruch 1, bei welcher das Drehteil (2) ein Antriebskraftübertragungsteil mit einer Kupplung zur Zuführung einer Drehantriebskraft aufweist,
wobei die Kupplung dann, wenn das von ihr übertragene Drehmoment einen vorbestimmten Wert übersteigt, durchrutscht und keine weitere Antriebskraft überträgt.

9. Zuführvorrichtung nach Anspruch 1, bei welcher das Drehteil (2) ein Antriebskraft-Übertragungsteil hat, welches eine Einwegkupplung zur Übertragung einer Drehantriebskraft enthält, und wobei dann, wenn ein von der Kupplung übertragenes Drehmoment der Richtung entgegengesetzt ist, in welcher der Pumpenschlauch mit Druck beaufschlagt wird, die Einwegkupplung daran gehindert wird, sich zu drehen oder durchzurutschen, und die Antriebskraft nicht übertragen kann.

## Revendications

1. Dispositif d'injection pour un supplément nutritionnel semi-solidifié qui injecte un supplément nutritionnel semi-solidifié à travers un tube de pompe (21) à partir d'un récipient de supplément nutritionnel rempli du supplément nutritionnel semi-solidifié, comprenant:
une base (1);
un rotateur (2) qui est supporté de façon rotative sur la base (1);
un rouleau (7) qui est supporté de façon rotative sur une périphérie extérieure du rotateur (2);
un élément de pressage (4) qui présente une surface de pressage en arc circulaire (4a) le long d'un chemin du rouleau (7) qui se déplace suivant une rotation du rotateur (2), qui est monté de façon mobile sur la base (1), et qui peut être placé de façon sélective dans une position rétractée, dans laquelle la surface de pressage (4a) est espacée à une distance du rotateur (2) de manière à former un espace en forme de rainure (8), et dans une position de proximité, dans laquelle la surface de pressage (4a) est plus proche du rotateur (2) que dans la position rétractée;
un couvercle (5) qui est monté de façon rotative sur la base (1) et qui peut être placé de façon sélective dans une position fermée, dans laquelle une région au-dessus de l'élément de pressage (4) et du rouleau (7) est fermée, et dans une position ouverte, dans laquelle cette région est ouverte; et
un élément de couplage (10) qui relie une rotation du couvercle (5) à un déplacement de l'élément de pressage (4), de telle sorte que lorsque le couvercle (5) se trouve dans la position ouverte, l'élément de pressage (4) se trouve dans la position rétractée, et lorsque le couvercle (5) se trouve dans la position fermée, l'élément de pressage (4) se trouve dans la position de proximité,
dans lequel le tube de pompe (21) peut être inséré dans l'espace en forme de rainure (8) lorsque l'élément de pressage (4) se trouve dans la position rétractée, et l'élément de pressage (4) est déplacé vers la position de proximité avec le tube de pompe (21) maintenu, et ensuite le rotateur (2) est mis en rotation, de telle sorte que le tube de pompe (21) soit entraîné par pression par le rouleau (7),
**caractérisé en ce que**:
un axe de rotateur (6) est prévu intégralement avec le rotateur (2) de manière à faire saillie à partir du corps de rotateur dans sa direction verticale,
un porte-rotateur (3) est prévu sur la base (1) et supporte de façon rotative des extrémités supérieure et inférieure de l'axe de rotateur (6), et
le couvercle (5) comporte un trou opérationnel (5a) qui coïncide avec la position de l'axe de rotateur (6) lorsque le couvercle (5) se trouve dans la position fermée, de telle sorte qu'une extrémité supérieure de l'axe de rotateur (6) passe à travers le trou opérationnel (5a) et soit exposée, permettant d'agencer une poignée (15) sur l'extrémité supérieure de l'axe de rotateur (6) à travers le trou opérationnel (5a), la poignée pouvant ainsi être manipulée pour faire tourner le rotateur (2).

2. Dispositif d'injection selon la revendication 1, comprenant en outre un support de base (1) pour supporter la base (1).

3. Dispositif d'injection selon la revendication 1, comprenant en outre un support de base pour supporter la base de telle sorte qu'elle ne soit pas instable lorsque le rotateur (2) est mis en rotation manuellement.

4. Dispositif d'injection selon l'une quelconque des revendications 1 à 3, comprenant en outre un compteur pour compter le nombre de rotations du rotateur.

5. Dispositif d'injection selon la revendication 4, dans lequel le compteur présente une structure qui est actionnée uniquement par un mécanisme.

6. Dispositif d'injection selon la revendication 1, dans lequel l'élément de pressage (4) comprend une partie avant qui présente la surface de pressage (35a) et une partie de support (37) qui est située au dos de la partie avant (35) avec un espace (36) interposé entre les deux et qui est connecté à la partie avant (35) par un élément de tampon (38a), et
l'élément de tampon (38a) rétracte la partie avant (35) par une déformation élastique lorsqu'une force de pressage qui dépasse une grandeur prédéterminée est appliquée par l'intermédiaire d'au moins la surface de pressage (35a).

7. Dispositif d'injection selon la revendication 1, dans lequel le rouleau (7) est supporté à la périphérie extérieure du rotateur (2) de telle sorte qu'un axe de rotation du rouleau (7) soit mobile dans une direction radiale du rotateur (2), et soit poussé en direction d'une circonférence extérieure du rotateur (2) par un élément de tampon (38b), et
l'élément de tampon (38b) rétracte le rouleau (7) en direction d'un centre du rotateur (2) par une déformation élastique lorsqu'une force de pressage qui dépasse une grandeur prédéterminée est appliquée par l'intermédiaire d'au moins la surface de pressage.

8. Dispositif d'injection selon la revendication 1, dans lequel le rotateur (2) comprend un élément de transmission de force d'entraînement qui comprend un embrayage pour recevoir une force d'entraînement rotative; et
lorsqu'un couple transmis par l'intermédiaire de l'embrayage est supérieur à une valeur prédéterminée, l'embrayage tourne fou et est incapable de transmettre la force d'entraînement.

9. Dispositif d'injection selon la revendication 1, dans lequel le rotateur (2) comprend un élément de transmission de force d'entraînement qui comprend un embrayage unidirectionnel pour recevoir une force d'entraînement rotative; et
lorsqu'un couple transmis par l'intermédiaire de l'embrayage unidirectionnel est orienté dans une direction opposée à une direction dans laquelle le tube de pompe est entraîné par pression, l'embrayage unidirectionnel est empêché de tourner, ou tourne fou et est incapable de transmettre la force d'entraînement.
